(19) **Europäisches Patentamt**
**European Patent Office**
**Office européen des brevets**

(11) **EP 1 847 268 A1**

(12) **EUROPEAN PATENT APPLICATION**
published in accordance with Art. 158(3) EPC

(43) Date of publication:
**24.10.2007 Bulletin 2007/43**

(21) Application number: **06712544.3**

(22) Date of filing: **30.01.2006**

(51) Int Cl.:
*A61K 31/4164* (2006.01)   *A61K 9/14* (2006.01)
*A61K 9/16* (2006.01)   *A61K 9/26* (2006.01)
*A61K 9/48* (2006.01)   *A61K 9/52* (2006.01)
*A61K 47/10* (2006.01)   *A61K 47/30* (2006.01)
*A61K 47/32* (2006.01)   *A61K 47/38* (2006.01)
*A61P 7/12* (2006.01)   *C07D 233/60* (2006.01)

(86) International application number:
**PCT/JP2006/301401**

(87) International publication number:
**WO 2006/080481 (03.08.2006 Gazette 2006/31)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **31.01.2005 JP 2005022797**

(71) Applicants:
• **KYORIN PHARMACEUTICAL CO., LTD.**
**Tokyo 101-8311 (JP)**
• **ONO PHARMACEUTICAL CO., LTD.**
**Osaka-shi, Osaka 541-8526 (JP)**

(72) Inventors:
• **NAKASHIMA, Katashi**
**3740016 (JP)**
• **AOKI, Yoshinobu**
**Tochigi, 3290112 (JP)**
• **KAZAMA, Kazuo**
**290114 (JP)**
• **ISHIZAKI, Toshihiro**
**i, 3290102 (JP)**

(74) Representative: **Vossius & Partner**
**Siebertstrasse 4**
**81675 München (DE)**

(54) **MULTIPLE UNIT ORAL SUSTAINED RELEASE PREPARATION AND PROCESS FOR PRODUCTION OF THE SAME**

(57)    A multiple-unit oral sustained release preparation is provided which allows controlled release of imidafenacin [4-(2-methyl-1-imidazolyl)-2,2-diphenylbutylamide]. The preparation serves to ensure a prolonged effect of imidafenacin and prevent rapid elevation in the blood levels of imidafenacin.

Specifically, granules or powders comprising imidafenacin dispersed in a water-insoluble polymer or a higher alcohol are used in the preparation. These preparations achieve sustained release of imidafenacin since the molecular network structure that the water-insoluble polymer or the higher alcohol forms during the preparation of the granules or powders serves to control the rate of diffusion of imidafenacin in water. Granules comprising a core granule having two layers of an inner imidafenacin coating and an outer water-insoluble polymer coating are used in the preparation. The water-insoluble polymer coating serves to control the rate of diffusion of imidafenacin in water and ensure sustained release of imidafenacin. The multiple-unit oral sustained release preparation is provided in the forms of capsules and tablets containing the granules or powders that allow controlled release of imidafenacin. The preparation achieves controlled release of imidafenacin over a prolonged period of time.

**EP 1 847 268 A1**

**Description**

TECHNICAL FIELD

The present invention relates to amultiple-unit oral sustained release preparation containing imidafenacin [4-(2-methyl-1-imidazolyl)-2,2-diphenylbutylamide] that is expected to be useful in the treatment of increased urinary frequency and urinary incontinence. The present invention also relates to a method for producing the same.

BACKGROUND ART

Urinary incontinence of elderly people has recently become an important public concern and significant efforts have been devoted to developing therapeutic products for increased urinary frequency and urinary incontinence. Imidafenacin, a novel compound developed by Kyorin Pharmaceutical Co., Ltd. (Patent Document 1), is a selective muscarinic antagonist and is considered as a medicinal candidate compound for increased urinary frequency and urinary incontinence (Non-Patent Document 1). An oral solid preparation has been disclosed as a dosage form of imidafenacin (Patent Document 2).
While imidafenacin is rapidly absorbed after ingestion, the drug has a relatively short half-life and requires multiple daily administrations by the conventional oral solid preparation.

[Patent Document 1] Japanese Patent Laid-Open Publication No. Hei 7-215943
[Patent Document 2] WO 01/34147 A1 pamphlet
[Non-Patent Document 1] Bioorg. Med. Chem., 1997. 7. 1151-116.

DISCLOSURE OF THE INVENTION

PROBLEMS TO BE SOLVED BY THE INVENTION

Sufferers of increased urinary frequency and urinary incontinence often have difficulty in leaving home for a long time or have anxiety that comfortable nighttime sleeping would be impaired. Thus, if a dosage form is developed that ensures a prolonged effect of a drug and can be taken less frequently, it would not only improve the quality of life of patients, but also help decrease the chance of patients forgetting to take the medication, thereby ensuring proper use of the drug. In addition, in case of dosage of medications having muscarine antagonistic activity ,they are known to cause side effects by rapid elevation in their blood levels. Thus, to prevent rapid elevation in their blood levels is considered as means for avoiding these side effects. Oral sustained release preparations that allow controlled release of a drug are divided into two major types: single-unit type and multiple-unit type. A typical oral sustained release preparation of single-unit type is tablets. Tablets are characteristic in that they gradually dissolve to release drugs in a sustained manner without disintegrating. In comparison, oral sustained release preparations of multiple-unit type are formed of small particles each capable of controlled release of a drug. Aside from granules, capsules and tablets may also be prepared as a multiple-unit type preparation. When drugs are administered by multiple-unit preparations, their absorption varies less and their release is highly reproducible. Further, it is known that drugs remain longer in the body when administered by multiple-unit preparations than when administered by single-unit preparations. For these reasons, multiple-unit preparations are considered more desirable dosage form as oral sustained release preparation. Accordingly, the present invention provides a multiple-unit oral sustained release preparation that is provided in the form of capsules or tablets containing granules or powder that allows control release of imidafenacin. The preparation of the present invention not only ensures a prolonged effect of imidafenacin, but also can prevents rapid elevation in the blood levels of imidafenacin.

MEANS TO BE SOLVE THE PROBLEM

The present inventors prepared granules and powders that allow controlled release of imidafenacin and used them to make capsules and tablets that served as the multiple-unit oral sustained release preparation of the present invention. Specifically, capsule preparations were made by filling capsules with the granules or powders and tablet preparations were made by mixing the granules or powders with pharmaceutically acceptable additives and compressing the mixture into tablets. The granules and powders were prepared by any of the following processes (1), (2) and (3):

(1) Imidafenacin, a water-insoluble polymer and pharmaceutically acceptable additives were kneaded together and the kneaded mixture was extruded into spherical particles, which were then dried.
(2) Imidafenacin was added to a heated and melted higher alcohol. The mixture was kneaded and cooled to harden. The hardened product was pulverized.

(3) Core granules were coated with imidafenacin and the coated granules were further coated with a water-insoluble polymer.

Accordingly, the present invention concerns the following:

1) A multiple-unit oral sustained release preparation, comprising a granule or powder that allows controlled release of imidafenacin, the granule or powder serving as a constituting unit,

2) The multiple-unit oral sustained release preparation according to 1), wherein the granule or powder comprises imidafenacin dispersed in a water-insoluble polymer and a pharmaceutically acceptable additive,

3) The multiple-unit oral sustained release preparation according to 1), wherein the granule or powder comprises imidafenacin dispersed in a higher alcohol,

4) The multiple-unit oral sustained release preparation according to 1), wherein the granule comprises a core granule, imidafenacin applied over the core granule, and a water-insoluble polymer applied over the imidafenacin,

5) The multiple-unit oral sustained release preparation according to 2) or 4), wherein the water-insoluble polymer is ethyl cellulose or aminoalkyl methacrylate copolymer RS,

6) The multiple-unit oral sustained release preparation according to 3), wherein the higher alcohol is stearyl alcohol,

7) The multiple-unit oral sustained release preparation according to 1) to 6), wherein the preparation is in the form of a capsule or a tablet,

8) A method for producing a multiple-unit oral sustained release preparation, comprising the steps of: kneading imidafenacin with a water-insoluble polymer; and forming it into granules,

9) A method for producing a multiple-unit oral sustained release preparation, comprising the steps of: coating a core granule with imidafenacin; and coating the imidafenacin-coated granule with a water-insoluble polymer,

10) The method for producing a multiple-unit oral sustained release preparation according to 8) or 9), wherein the water-insoluble polymer is ethyl cellulose or aminoalkyl methacrylate copolymer RS,

11) A method for producing a multiple-unit oral sustained release preparation, comprising the steps of: adding imidafenacin to a higher alcohol which is molten by heating, and kneading it; cooling the kneadedmixture to harden it; and pulverizing the hardened mixture,

12) The method for producing a multiple-unit oral sustained release preparation according to 11), wherein the higher alcohol is stearyl alcohol.

EFFECTS OF THE INVENTION

The granules or powders comprising imidafenacin dispersed in a water-insoluble polymer or a higher alcohol achieve sustained release of imidafenacin since the molecular network structure that the water-insoluble polymer or the higher alcohol forms during the preparation of the granules or powders serves to control the rate of diffusion of imidafenacin in water. Likewise, the granules comprising a core granule having two layers of an inner imidafenacin coating and an outer water-insoluble polymer coating achieve sustained release of imidafenacin since the water-insoluble polymer coating serves to control the rate of diffusion of imidafenacin in water. Capsules or tablets containing the granules or powders that allow controlled release of imidafenacin also allow controlled release of imidafenacin over a prolonged period of time and continuously.

BRIEF DESCRIPTION OF THE DRAWINGS

Fig. 1 is a graph showing the dissolution curves for Examples 1, 2 and 3 and Comparative Example.

Fig. 2 is a graph showing the dissolution curves for Examples 4, 5 and 6 and Comparative Example.

Fig. 3 is a graph showing the dissolution curves for Examples 7, 8 and 9 and Comparative Example.

Fig. 4 is a graph showing the dissolution curves for Examples 10, 11 and 12 and Comparative Example.

Fig. 5 is a graph showing the gastrointestinal absorption property of imidafenacin in rats obtained in Experiment Example 2. Each number indicates average $\pm$ SD for n = 3.

BEST MODE FOR CARRYING OUT THE INVENTION

Imidafenacin, the active ingredient of the multiple-unit oral sustained release preparation of the present invention, also known as 4-(2-methyl-1-imidazolyl)-2,2-diphenylbutylamide, is an anticholinergic agent selective for the bladder and is effective in the treatment of increased urinary frequency and urinary incontinence.

The multiple-unit oral sustained release preparation of the present invention is characterized in that it uses granules or powders, as a constituting unit, that allows controlled release of imidafenacin.

Specifically, the multiple-unit oral sustained release preparation of the present invention is constituted by either granules or powders comprising imidafenacin dispersed in a water-insoluble polymer or a higher alcohol, or granules comprising a core granule having two layers of an inner imidafenacin coating and an outer water-insoluble polymer coating.

The water-insoluble polymer for use in the multiple-unit oral sustained release preparation of the present invention is a polymer material that can form a water-insoluble polymer network (matrix) structure or a strong dense membrane. Such polymers are commonly used in pharmaceutical products as a sustained release base, enteric-coated base or gastric-coated base. Examples of the water-insoluble polymer for use in the present invention include purified shellac, white shellac, carboxymethylethylcellulose, hydroxypropyl methylcellulose acetate phthalate, hydroxymethylcellulose acetate succinate, cellulose acetate phthalate, ethyl cellulose and aminoalkyl methacrylate copolymer RS. Of these, ethyl cellulose and aminoalkyl methacrylate copolymer RS are particularly suitable for use in the present invention.

Ethyl cellulose is commercially available from Dow Chemical Company under the trade name of ETHOCEL. The product is available in different particle sizes and viscosities. Ethyl cellulose with average viscosity of 7 or 10cps is particularly suitable for use in the present invention.

Aminoalkyl methacrylate copolymer RS is commercially available from Degussa under the trade name of EUDRAGIT RS100.

The higher alcohol for use in the multiple-unit oral sustained release preparation of the present invention is a material that melts at 100°C or a lower temperature, and can dissolve or disperse a drug in it upon melting and can form a water-insoluble molecular network (matrix) when cooled and hardened. Such higher alcohols are commonly used in pharmaceutical products as an ointment base or sustained release base. Examples of the higher alcohol for use in the present invention include cetanol and stearyl alcohol. Of these, stearyl alcohol is more suitable for use in the present invention.

The granules of the present invention comprising imidafenacin dispersed in the above-described water-insoluble polymer can be obtained by kneading the water-insoluble polymer with imidafenacin by a known kneading means, such as MECHANOMILL, and extruding the kneaded mixture into granules. Further, the granules may be pulverized to form a powder comprising imidafenacin dispersed in the water-insoluble polymer.

The granules or powders of the present invention comprising imidafenacin dispersed in the above-described higher alcohol can be obtained by adding imidafenacin to a higher alcohol molten by heating, kneading them, cooling the kneaded mixture to harden it, and forming or crashing the hardened mixture.

The granules comprising a core granule having two layers of an inner imidafenacin coating and an outer water-insoluble polymer coating can be obtained, for example, by spraying a solution of imidafenacin in an ethanol/water mixture onto the core granule to coat the core granule with imidafenacin, and further coating the imidafenacin-coated granule with the water-insoluble polymer.

The core granule for use in the multiple-unit oral sustained release preparation of the present invention is a granule having a shape, size and composition suitable for imidafenacin coating. For example, the core granule may be a spherical particle made of crystalline cellulose, a spherical granule made of purified sucrose, a spherical granule made of a mixture of purified sucrose and starch, or spherical particle made of a mixture of lactose and crystalline cellulose. Spherical granules made of lactose, crystalline cellulose and polyvinylpyrrolidone are particularly preferred for use in the present invention.

The multiple-unit oral sustained release preparation of the present invention may contain any pharmaceutically acceptable additive. Examples of the pharmaceutically acceptable additive include excipients, binders, disintegrating agents, coating aids, lubricants and capsule shells that are commonly used in the production of pharmaceutical products. Examples of the excipient include sugars such as lactose and glucose, sugar alcohols such as D-sorbitol and mannitol, celluloses such as crystalline cellulose, and starches such as corn starch and partly pregelatinized starch. Lactose and crystalline cellulose are particularly suitable for use in the present invention. Examples of the binder include crystalline cellulose, sugars, dextrin, hydroxypropylcellulose, hydroxypropylmethylcellulose, polyvinylpyrrolidone, macrogol, powdered acacia, gelatin and pullulan. Of these, hydroxypropylcellulose and polyvinylpyrrolidone are particularly suitable for use in the present invention. Examples of the disintegrating agent include corn starch, carboxymethylcellulose, carboxymethylcellulose calcium, low-substituted hydroxypropylcellulose, croscarmellose sodium, carboxymethylstarch sodium and crosslinked polyvinylpyrrolidone. Of these, croscarmellose sodium is particularly suitable for use in the present invention. Examples of the coating aid include hydrogenated oil, stearic acid, cetanol, medium-chain fatty acid triglyceride, triacetin, polyethylene glycol and triethyl citrate. Of these, polyethylene glycol and triethyl citrate are particularly preferred for use in the present invention. Examples of the lubricant include magnesium stearate, calcium stearate, talc and hydrogenated oil. Of these, magnesium stearate is particularly preferred for use in the present invention. Examples of the capsule shell include gelatin, hydroxypropylmethylcellulose and pullulan. If necessary, other pharmaceutically acceptable additives may also be added to the multiple-unit oral sustained release preparation of the present invention.

The multiple-unit oral sustained release preparation of the present invention is preferably provided in the dosage forms of capsules and tablets.

The capsules may be produced by using any suitable technique. For example, granules or powders containing imidafenacin may be filled in capsules either directly or as a mixture with various pharmaceutically acceptable additives.

The tablets may be produced by using any suitable technique. For example, granules or powders containing imidafenacin may be compressed into tablets either directly or as a mixture with various pharmaceutically acceptable additives.

If necessary, the compressed tablets may be coated with a film by using common techniques. Any coating agent commonly used in pharmaceutical products may be used for this purpose.

The present invention will now be described with reference to examples, which are not intended to limit the scope of the present invention in any way.

Example 1

1.0 g of imidafenacin, 195.0 g of ethyl cellulose (trade name : ETHOCEL (Standard 10cps Premium) Dow Chemical Company), 51.0 g of crystalline cellulose (trade name: AVICEL PH-101, Asahi Kasei Chemicals Corporation) and 12.0 g of hydroxypropylcellulose (trade name: HPC L, Nippon Soda Co., Ltd.) were placed in a Mechanomill (trade name: MECHANOMILL MM-10N, Okada Seiko Co., Ltd.) and were mixed for 10 min at 400 rpm. 19.4 g of triethyl citrate (trade name: Citroflex 2 (SC-60) Morimura Shoji Co., Ltd.) was then added and the mixture was further stirred for 2 min at 400 rpm. Subsequently, water and ethanol (95) were added in appropriate amounts and the mixture was kneaded for 1 min at 400 rpm. Using a Domegran (trade name: DOMEGRAN DG-L1, equipped with a 0.7$\phi$·0.7T screen, Fuji Paudal Co. , Ltd.), the kneaded product was extruded at a screw revolution number of 20 rpm. The extrusion was then formed into spheres on a Mechanomill equipped with a rotary disc (crosshatch type) for 10 min at 1000 rpm. The resulting spheres were dried at 50°C for 120 min on an air drier (trade name: AIR DRIER 30C, Fuji Paudal Co. , Ltd.). The dried product was sieved through a 850$\mu$m-aperture stainless sieve. To 40 g of the sieved product, 0.23 g of hydrated silicon dioxide (trade name: CARPLEX #67, Shionogi Pharmaceutical Co., Ltd.) was added and 140 mg of this mixture was filled in hydroxypropylmethylcellulose capsules (trade name: QUALI-V, Shionogi Qualicaps Co. , Ltd.). This gave capsules each containing 0.5 mg imidafenacin.

Example 2

0.5 g of imidafenacin, 195.0 g of ethyl cellulose (trade name: ETHOCEL (Standard 10cps Premium) Dow Chemical Company), 25.5 g of crystalline cellulose (trade name: AVICEL PH-101, Asahi Kasei Chemicals Corporation) and 6.0 g of hydroxypropylcellulose (trade name: HPC L, Nippon Soda Co., Ltd.) were placed in a Mechanomill (trade name: MECHANOMILL MM-10N, Okada Seiko Co., Ltd.) and were mixed for 10 min at 400 rpm. 9.7 g of triethyl citrate (trade name: Citroflex 2 (SC-60), Morimura Shoji Co., Ltd.) was then added and the mixture was further stirred for 2 min at 400 rpm. Subsequently, water and ethanol (95) were added and the mixture was kneaded for 1 min at 400 rpm. Using a Domegran (trade name: DOMEGRAN DG-L1, equipped with a 1. 0$\phi$·1. 0T screen, Fuji Paudal Co., Ltd.), the kneaded product was extruded at a screw revolution number of 20 rpm. The extrusion was then formed into spheres on a Mechanomill equipped with a rotary disc (crosshatch type) for 5 min at 1000 rpm. The resulting spheres were dried at 50°C for 120 min on an air drier (trade name: AIR DRIER 30C, Fuji Paudal Co. , Ltd.). The dried product was sieved through a 1400$\mu$m-apreture stainless sieve and the sieved product was further sieved through a 850$\mu$m-aperture stainless sieve. To 40 g of the granules remaining on the 850$\mu$m-aperture stainless sieve, 0.135 g of hydrated silicon dioxide (trade name: CARPLEX #67, Shionogi Pharmaceutical Co., Ltd.) was added and 237.5 mg of this mixture was filled in hydroxypropylmethylcellulose capsules (trade name: QUALI-V, Shionogi Qualicaps Co., Ltd.). This gave capsules each containing 0.5 mg imidafenacin.

Example 3

45 g of stearyl alcohol (trade name: STEARYL ALCOHOL NAA-45, NOF Corporation) was stirred and melted in a water bath at approximately 90°C. To the molten alcohol, 5 g of imidafenacin was added and the mixture was stirred to disperse imidafenacin uniformly. The mixture was transferred to a stainless tub to allow the mixture to quickly spread over the floor of the tub. The spread product was left at room temperature for approximately 24 hours. Once hardened completely, the product was scraped off the floor by a spatula and thoroughly pulverized in a mortar. 10 mg of the pulverized product was filled in hydroxypropylmethylcellulose capsules (trade name: QUALI-V, Shionogi Qualicaps Co., Ltd.). This gave capsules each containing 0.5 mg imidafenacin.

Example 4

700 g of spherical granules composed of lactose, crystalline cellulose and polyvinylpyrrolidone (to serve as core granules) was placed in a tumbling fluidized bed granulator (trade name: NEW MARUMERIZER NQ-160, Fuji Paudal Co., Ltd.). A solution of imidafenacin in a 1:1 mixture of ethanol (95) and water was then sprayed onto the granules to form base granules. 50.5 mg of the base granules was composed of 50 mg of spherical granules of lactose/crystalline cellulose and 0.5mg of imidafenacin. 700g of the above base granules was placed in a tumbling fluidized bed granulator and was coated with a sustained release coating solution having a composition shown below to form 56. 08 mg of sustained release granules. The resulting sustained release granules were filled in hydroxypropylmethylcellulose capsules (trade name: QUALI-V, Shionogi Qualicaps Co., Ltd.) to give capsules each containing 0.5 mg imidafenacin.

Composition of sustained release coating solution:

| EUDRAGIT RS100 | 6.93 mg |
| Polyethylene glycol 6000 | 0.43 mg |
| Sodium chloride | 0.22 mg |
| Ethanol (95) | 46.52 mg |
| Purified water | 18.08 mg |
| Total | 7.58 mg (solid component) |

Example 5

700 g of spherical granules composed of lactose, crystalline cellulose and polyvinylpyrrolidone (to serve as core granules) was placed in a tumbling fluidized bed granulator (trade name: NEW MARUMERIZER NQ-160, Fuji Paudal Co., Ltd.). A solution of imidafenacin in a 1:1 mixture of ethanol (95) and water was then sprayed onto the granules to form base granules. 50.5 mg of the base granules was composed of 50 mg of spherical granules of lactose/crystalline cellulose and 0.5 mg of imidafenacin. 700 g of the base granules was placed in a tumbling fluidized bed granulator and was coated with a sustained release coating solution having a composition shown below to form 63.12 mg of sustained release granules. The resulting sustained release granules were filled in hydroxypropylmethylcellulose capsules (trade name: QUALI-V, Shionogi Qualicaps Co., Ltd.) to give capsules each containing 0.5 mg imidafenacin.

Composition of sustained release coating solution:

| EUDRAGIT RS100 | 1.54 mg |
| Polyethylene glycol 6000 | 0.72 mg |
| Sodium chloride | 0.36 mg |
| Ethanol (95) | 77.47 mg |
| Purified water | 30.12 mg |
| Total | 12.62 mg (solid component) |

Example 6

700 g of spherical granules composed of lactose, crystalline cellulose and polyvinylpyrrolidone (to serve as core granules) was placed in a tumbling fluidized bed granulator (trade name: NEW MARUMERIZER NQ-160, Fuji Paudal Co., Ltd.). A solution of imidafenacin in a 1:1 mixture of ethanol (95) and water was then sprayed onto the granules to form base granules. 50.5 mg of the base granules was composed of 50 mg of spherical granules of lactose/crystalline cellulose and 0.5 mg of imidafenacin. 700 g of the base granules was placed in a tumbling fluidized bed granulator and was coated with a sustained release coating solution having a composition shown below to form 65.15 mg of sustained release granules. The resulting sustained release granules were filled in hydroxypropylmethylcellulose capsules (trade name: QUALI-V, Shionogi Qualicaps Co. , Ltd.) to give capsules each containing 0.5 mg imidafenacin.

Composition of sustained release coating solution:

| | |
|---|---|
| EUDRAGIT RS100 | 13.39 mg |
| Polyethylene glycol 6000 | 0.84 mg |
| Sodium chloride | 0.42 mg |
| Ethanol (95) | 89.88 mg |
| Purified water | 34.93 mg |
| Total | 14.65 mg (solid component) |

Example 7

700 g of spherical granules composed of lactose, crystalline cellulose and polyvinylpyrrolidone (to serve as core granules) was placed in a tumbling fluidized bed granulator (trade name: NEW MARUMERIZER NQ-160, Fuji Paudal Co., Ltd.). A solution of imidafenacin in a 1:1 mixture of ethanol (95) and water was then sprayed onto the granules to form base granules. 50.5 mg of the base granules was composed of 50 mg of spherical granules of lactose/crystalline cellulose and 0.5 mg of imidafenacin. 700 g of the base granules was placed in a tumbling fluidized bed granulator and was coated with a sustained release coating solution having a composition shown below to form 52.51 mg of sustained release granules. The resulting sustained release granules were filled in hydroxypropylmethylcellulose capsules (trade name: QUALI-V, Shionogi Qualicaps Co. , Ltd.) to give capsules each containing 0. 5 mg imidafenacin.

Composition of sustained release coating solution:

| | |
|---|---|
| ETHOCEL (Std 7cps premium) | 1.86 mg |
| CITROFLEX 2 (SC-60) | 0.10 mg |
| Sodium chloride | 0.05 mg |
| Ethanol (95) | 40.59 mg |
| Purified water | 9.06 mg |
| Total | 2.01 mg (solid component) |

Example 8

700 g of spherical granules composed of lactose, crystalline cellulose and polyvinylpyrrolidone (to serve as core granules) was placed in a tumbling fluidized bed granulator (trade name: NEW MARUMERIZER NQ-160, Fuji Paudal Co., Ltd.). A solution of imidafenacin in a 1:1 mixture of ethanol (95) and water was then sprayed onto the granules to form base granules. 50.5 mg of the base granules was composed of 50 mg of spherical granules of lactose/crystalline cellulose and 0.5 mg of imidafenacin. 700 g of the base granules was placed in a tumbling fluidized bed granulator and was coated with a sustained release coating solution having a composition shown below to form 53.02 mg of sustained release granules. The resulting sustained release granules were filled in hydroxypropylmethylcellulose capsules (trade name: QUALI-V, Shionogi Qualicaps Co., Ltd.) to give capsules each containing 0.5 mg imidafenacin.

Composition of sustained release coating solution:

| | |
|---|---|
| ETHOCEL (Std 7cps premium) | 2.33 mg |
| CITROFLEX 2 (SC-60) | 0.13 mg |
| Sodium chloride | 0.06 mg |
| Ethanol (95) | 50.85 mg |
| Purified water | 11.35 mg |
| Total | 2.52 mg (solid component) |

Example 9

700 g of spherical granules composed of lactose, crystalline cellulose and polyvinylpyrrolidone (to serve as core granules) was placed in a tumbling fluidized bed granulator (trade name: NEW MARUMERIZER NQ-160, Fuji Paudal Co., Ltd.). A solution of imidafenacin in a 1:1 mixture of ethanol (95) and water was then sprayed onto the granules to form base granules. 50.5 mg of the base granules was composed of 50 mg of spherical granules of lactose/crystalline cellulose and 0.5 mg of imidafenacin. 700 g of the base granules was placed in a tumbling fluidized bed granulator and was coated with a sustained release coating solution having a composition shown below to form 53.54 mg of sustained release granules. The resulting sustained release granules were filled in hydroxypropylmethylcellulose capsules (trade name: QUALI-V, Shionogi Qualicaps Co. , Ltd.) to give capsules each containing 0. 5 mg imidafenacin.

Composition of sustained release coating solution:

| | |
|---|---|
| ETHOCEL (Std 7cps premium) | 2.80 mg |
| CITROFLEX 2 (SC-60) | 0.16 mg |
| Sodium chloride | 0.08 mg |
| Ethanol (95) | 62.41 mg |
| Purified water | 13.93 mg |
| Total | 3.04 mg (solid component) |

Example 10

0.5 g of imidafenacin, 195. 0 g of ethyl cellulose (trade name: ETHOCEL (Standard 10cps Premium) Dow Chemical Company), 25.5 g of crystalline cellulose (trade name: AVICEL PH-101, Asahi Kasei Chemicals Corporation) and 6.0 g of hydroxypropylcellulose (trade name: HPC L, Nippon Soda Co., Ltd.) were placed in a Mechanomill (trade name: MECHANOMILL MM-10N, Okada Seiko Co., Ltd.) and were mixed for 10 min at 400 rpm. 9.7 g of triethyl citrate (trade name: Citroflex 2 (SC-60), Morimura Shoji Co., Ltd.) was then added and the mixture was further stirred for 2 min at 400 rpm. Subsequently, water and ethanol (95) were added and the mixture was kneaded for 1 min at 400 rpm. Using a Domegran (trade name: DOMEGRAN DG-L1, equipped with a 1.0ϕ·1.0T screen, Fuji Paudal Co. , Ltd.), the kneaded product was extruded at a screw revolution number of 20 rpm. The extrusion was then formed into spheres on a Mechanomill equipped with a rotary disc (crosshatch type) for 5 min at 1000 rpm. The resulting spheres were dried at 50°C for 120 min on an air drier (trade name: AIR DRIER 30C, Fuji Paudal Co., Ltd.). The dried product was sieved through a 850$\mu$m meshstainless sieve and the sieved product was further sieved through a 590$\mu$m-apreture stainless sieve. To 40 g of the granules remaining on the 590$\mu$m mesh stainless sieve, 0. 135 g of hydrated silicon dioxide (trade name: CARPLEX #67, Shionogi Pharmaceutical Co., Ltd.) was added to form sustained release granules. On the other hand, 162.5 g of direct-tabletting lactose (trade name: DILACTOSE S, Freund Corporation) was mixed with 0.4 g of magnesium stearate in a bag to form a mixed powder. Using a single-action tabletting machine with a 9.5 mmϕ, 12R punch, 23.75 g of the mixed powder and 16.25 g of the sustained release granules were compressed into tablets each weighing 400 mg and containing 0.5 mg imidafenacin.

Example 11

700 g of spherical granules composed of lactose, crystalline cellulose and polyvinylpyrrolidone (to serve as core granules) was placed in a tumbling fluidized bed granulator (trade name: NEW MARUMERIZER NQ-160, Fuji Paudal Co., Ltd.). A solution of imidafenacin in a 1:1 mixture of ethanol (95) and water was then sprayed onto the granules to form base granules. 50.5 mg of the base granules was composed of 50 mg of spherical granules of lactose/crystalline cellulose and 0.5 mg of imidafenacin. 700 g of the base granules was placed in a tumbling fluidized bed granulator and was coated with a sustained release coating solution having a composition shown below to form 58.08 mg of sustained release granules. On the other hand, 81.22 g of direct-tableting lactose (trade name DILACTOSES, Freund Corporation), 38.00g of crystalline cellulose (trade name: CEOLUS KG-802, Asahi Kasei Chemicals Corporation), 12.70 g of croscarmellose sodium (trade name: Ac-Di-Sol SD-711, FMC Corporation), 0.19 g of hydrated silicon dioxide (trade name: CARPLEX#67, Shionogi Pharmaceutical Co., Ltd.) and 0.19 g of magnesium stearate were mixed together in a bag to form a mixed powder. Using a single-action tableting machine with a 7.5 mmϕ, 9R punch, 131.92 g of the mixed powder and 58.08 g of the sustained release granules were compressed into tablets each weighing 190 mg and containing 0.5 mg imidafenacin.

Composition of sustained release coating solution:

| | |
|---|---|
| EUDRAGIT RS100 | 6.93 mg |
| Polyethylene glycol 6000 | 0.43 mg |
| Sodium chloride | 0.22 mg |
| Ethanol (95) | 46.52 mg |
| Purified water | 18.08 mg |
| Total | 7.58 mg (solid component) |

Example 12

700 g of spherical granules composed of lactose, crystalline cellulose and polyvinylpyrrolidone (to serve as core granules) was placed in a tumbling fluidized bed granulator (trade name: NEW MARCUMERIZER NQ-160, Fuji Paudal Co. , Ltd.). A solution of imidafenacin in a 1:1 mixture of ethanol (95) and water was then sprayed onto the granules to form base granules. 50.5 mg of the base granules was composed of 50 mg of spherical granules of lactose/crystalline cellulose and 0.5 mg of imidafenacin. 700 g of the base granules was placed in a tumbling fluidized bed granulator and was coated with a sustained release coating solution having a composition shown below to form 53.54 mg of sustained release granules. On the other hand, 79. 91 g of crystalline cellulose (trade name: AVICEL PH-302, Asahi Kasei Chemicals Corporation), 45.30 g of crystalline cellulose (trade name: CEOLUS KG-802, Asahi Kasei Chemicals Corporation), 11.25 g of croscarmellose sodium (trade name: Ac-Di-Sol SD-711, FMC Corporation), 0.19 g of hydrated silicon dioxide (trade name: CARPLEX#67, Shionogi Pharmaceutical Co., Ltd.) and 0.19 g of magnesium stearate were mixed together in a bag to form a mixed powder. Using a single-action tabletting machine with a 7.5 mm$\phi$, 9R punch, 136.46 g of the mixed powder and 53.54 g of the sustained release granules were compressed into tablets each weighing 190 mg and containing 0.5 mg imidafenacin.

Composition of sustained release coating solution:

| | |
|---|---|
| ETHOCEL (Std 7cps premium) | 2.80 mg |
| CITROFLEX 2 (SC-60) | 0.16 mg |
| Sodium chloride | 0.08 mg |
| Ethanol (95) | 62.41 mg |
| Purified water | 13.93 mg |
| Total | 3.04 mg (solid component) |

Comparative Example

243.2 g of partly pregelatinized starch (trade name: STARCH 1500G, Colorcon Inc.) and 970.8 g of crystalline cellulose (trade name: AVICEL PH-301, Asahi Kasei Chemicals Corporation) were placed in a flow coater FBG-5 (Freund Corporation). A solution of 2 g imidafenacin and 12.8 g polyvinylpyrrolidone (trade name: POVIDONE, BASF Co., Ltd.) in a 1: 1 mixture of ethanol (95) and water was then sprayed to form granules. The granules were sieved through a 850$\mu$m mesh sieve and 3.2 g of magnesium stearate was added to the sorted granules to obtain granules for tabletting. Using a rotary tabletting machine HT-P18SSII (Hata Iron Works Co., Ltd.), the granules were compressed into simple tablets with a diameter of 7.5 mm and a weight of 154 mg. The resulting tablets were coated with OPADRY 03A45009 (Colorcon Inc.) (6 mg per tablet), and a small amount of carnauba wax (trade name: POLISHING WAX 103, Freund Corporation) was added to make film-coated tablets each containing 0.25 mg imidafenacin.

Experiment Example 1

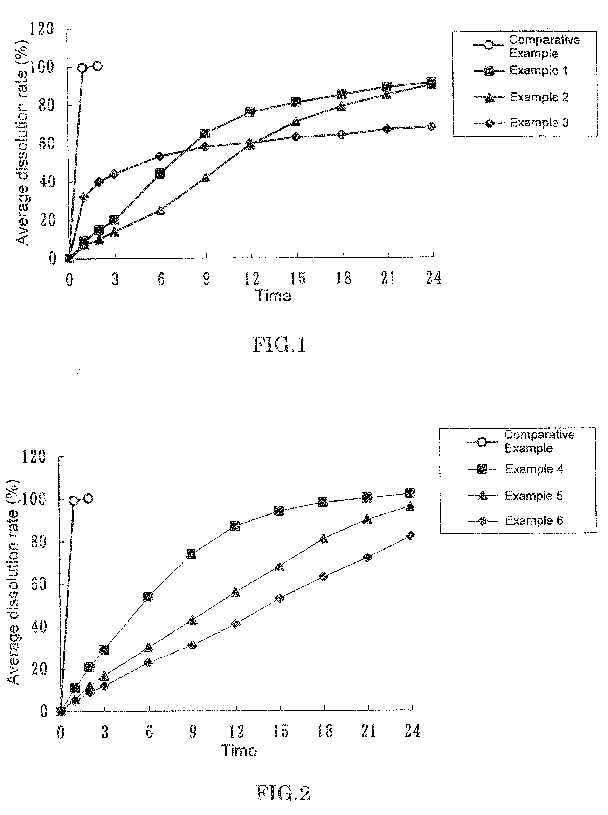

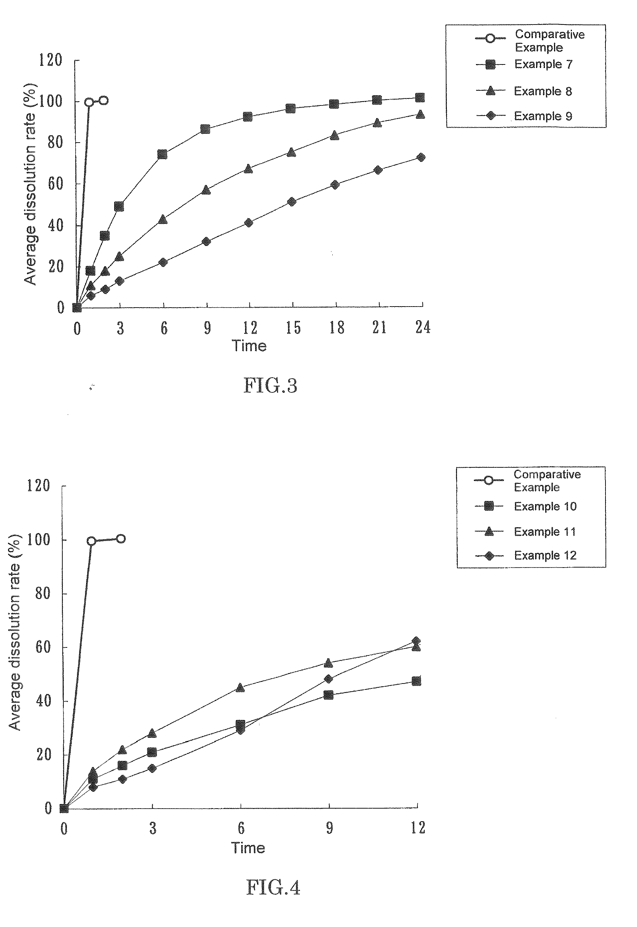

According to the method 2 of the dissolution test described in Japanese Pharmacopoeia Fourteenth Edition, the preparations of Examples 1 through 12 and Comparative Example were each tested at 50 rpm in 900 mL purified water at 37°C (n = 6 for Comparative Example and n = 3 for all Examples. A sinker was used). The results are shown in Figs. 1 through 4.

As can be seen from the figures, each of the preparations of Examples 1 through 12 showed sustained drug release properties as compared to the preparation of Comparative Example.

Experiment 2

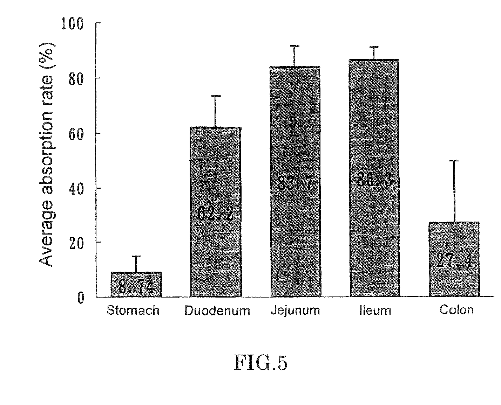

In designing oral sustained release preparations of imidafenacin, it is necessary to know the absorption properties of imidafenacin in the gastrointestinal tract. We therefore examined the gastrointestinal absorption properties of imidafenacin in rats using the *in-situ* loop technique. The specific procedures were as follows: Male Wistar rats (aged 7 to 9 weeks) were dissected under anesthesia with pentobarbital sodium and the common bile duct was ligated. Loops were prepared from the stomach, duodenum, jejunum, ileum and colon. 0.2 mL each of an isotonic solution (500 μg/mL) of imidafenacin was injected into the loops in tripricate. After 1 hour, the lumen of the loops was washed sequentially with water and 0.01 mol/L hydrochloric acid and the wash was collected from each loop. The concentration of imidafenacin in the wash was determined by high performance liquid chromatography. The standard curve was determined by applying the least squares method to the ratio of the peak areas between the imidafenacin concentration for a similarly treated standard specimen and the concentration of internal standard of imidafenacin determined by chromatography. The concentration of imidafenacin in the wash for a specimen was determined by substituting the ratio of the peak area into the standard curve. The absorption of imidafenacin for each loop was calculated by the equations (1) and (2) below. The results are shown in Fig. 5.

```
% Remaining imidafenacin in a loop = (Conc. of imidafenacin in the wash for the loop) x 25        (1)
```

```
% Absorption of imidafenacin in a loop = 100 - % Remaining imidafenacin

in that loop      (2)
```

The results indicate that imidafenacin was effectively absorbed in any part of the small intestine while the absorption was less significant in the stomach. Some absorption was also observed in colon.

INDUSTRIAL APPLICABILITY

As set forth, the present invention provides capsules and tablets that allow controlled release of imidafenacin. Analysis of the gastrointestinal absorption properties of imidafenacin in rats indicates that the compound is effectively absorbed in any part of the small intestine with some being absorbed in colon. The results suggest that a similar absorption pattern is likely to be observed in humans. Studies have shown that the multiple-unit oral sustained release preparation, when administered to human patients, passes the stomach and small intestine in 5 to 6 hours and subsequently remains in the colon for as long as 20 hours. This observation suggests that the capsules and tablets of the present invention, prepared as multiple-unit oral sustained release preparations that allow sustained release of imidafenacin, can serve to effectively prevent the rapid elevation in the blood levels of imidafenacin.

**Claims**

1.   A multiple-unit oral sustained release preparation, comprising a granule or powder that allows controlled release of imidafenacin, the granule or powder serving as a constituting unit.

2.   The multiple-unit oral sustained release preparation according to claim 1, wherein the granule or powder comprises imidafenacin dispersed in a water-insoluble polymer and a pharmaceutically acceptable additive.

3.   The multiple-unit oral sustained release preparation according to claim 1, wherein the granule or powder comprises imidafenacin dispersed in a higher alcohol.

4.   The multiple-unit oral sustained release preparation according to claim 1, wherein the granule comprises a core granule, imidafenacin applied over the core granule, and a water-insoluble polymer applied over the imidafenacin.

5. The multiple-unit oral sustained release preparation according to claim 2 or 4, wherein the water-insoluble polymer is ethyl cellulose or aminoalkyl methacrylate copolymer RS.

6. The multiple-unit oral sustained release preparation according to claim 3, wherein the higher alcohol is stearyl alcohol.

7. The multiple-unit oral sustained release preparation according to any one of claims 1 to 6, wherein the preparation is in the form of a capsule or a tablet.

8. A method for producing a multiple-unit oral sustained release preparation, comprising the steps of: kneading imidafenacin with a water-insoluble polymer; and forming it into granules.

9. A method for producing a multiple-unit oral sustained release preparation, comprising the steps of: coating a core granule with imidafenacin; and coating the imidafenacin-coated granule with a water-insoluble polymer.

10. The method for producing a multiple-unit oral sustained release preparation according to claim 8 or 9, wherein the water-insoluble polymer is ethyl cellulose or aminoalkyl methacrylate copolymer RS.

11. A method for producing a multiple-unit oral sustained release preparation, comprising the steps of: adding imidafenacin to a higher alcohol which is molten by heating, and kneading it; cooling the kneaded mixture to harden it; and pulverizing the hardened mixture.

12. The method for producing a multiple-unit oral sustained release preparation according to claim 11, wherein the higher alcohol is stearyl alcohol.

FIG.1

FIG.2

FIG.3

FIG.4

FIG.5

**INTERNATIONAL SEARCH REPORT**

| International application No. |
| --- |
| PCT/JP2006/301401 |

A. CLASSIFICATION OF SUBJECT MATTER
*A61K31/4164*(2006.01), *A61K9/14*(2006.01), *A61K9/16*(2006.01), *A61K9/26*
(2006.01), *A61K9/48*(2006.01), *A61K9/52*(2006.01), *A61K47/10*(2006.01),
*A61K47/30*(2006.01), *A61K47/32*(2006.01), *A61K47/38*(2006.01),
According to International Patent Classification (IPC) or to both national classification and IPC

B. FIELDS SEARCHED

Minimum documentation searched (classification system followed by classification symbols)
A61K9/14, A61K9/16, A61K9/26, A61K9/48, A61K9/52, A61K31/4164, A61K47/10,
A61K47/30, A61K47/32, A61K47/38, A61P7/12, C07D233/60

Documentation searched other than minimum documentation to the extent that such documents are included in the fields searched
Jitsuyo Shinan Koho        1922-1996    Jitsuyo Shinan Toroku Koho   1996-2006
Kokai Jitsuyo Shinan Koho  1971-2006    Toroku Jitsuyo Shinan Koho   1994-2006

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| Y | WO 2001/034147 A1 (Kyorin Pharmaceutical Co., Ltd.), 17 May, 2001 (17.05.01), Full text & EP 1245232 A1          & JP 2001-536145 A | 1-12 |
| Y | JP 6-009388 A (Kodama Kabushiki Kaisha), 18 January, 1994 (18.01.94), Full text; particularly, Par. Nos. [0003], [0010] (Family: none) | 1-12 |
| Y | JP 5-339151 A (Kodama Kabushiki Kaisha), 21 December, 1993 (21.12.93), Full text (Family: none) | 1-12 |

☒ Further documents are listed in the continuation of Box C.      ☐ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 26 April, 2006 (26.04.06) | 16 May, 2006 (16.05.06) |

| Name and mailing address of the ISA/ | Authorized officer |
| --- | --- |
| Japanese Patent Office | |
| Facsimile No. | Telephone No. |

Form PCT/ISA/210 (second sheet) (April 2005)

**INTERNATIONAL SEARCH REPORT**

| International application No. |
|---|
| PCT/JP2006/301401 |

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2001/070221 A1 (Nippon Shinyaku Co., Ltd.), 27 September, 2001 (27.09.01), Full text & EP 1283040 A1 & US 2003/0044465 A1 & JP 2001-568419 A | 1-12 |
| Y | JP 63-154619 A (Kowa Yakuhin Kogyo Kabushiki Kaisha), 27 June, 1988 (27.06.88), Full text (Family: none) | 1-12 |
| Y | JP 63-243030 A (SSP Co., Ltd.), 07 October, 1988 (07.10.88), Full text (Family: none) | 1-3,5-8, 10-12 |
| Y | WO 2004/050075 A1 (LAB DEL ESTEVE S.A.), 17 June, 2004 (17.06.04), Full text & EP 1596850 A1 | 1-3,5-8, 10-12 |
| Y | JP 11-124327 A (Euro-Celtique S.A.), 11 May, 1999 (11.05.99), Full text & EP 624366 A1 & JP 7-149648 A & US 5591452 A & JP 7-053361 A & US 6143328 A & US 6254887 B1 & US 2001/0036477 A1 & JP 2002-154954 A | 1-3,5-8, 10-12 |
| Y | JP 63-122623 A (Euro-Celtique S.A.), 26 May, 1988 (26.05.88), Full text & EP 271193 A & US 4844909 A & US 4990341 A | 1-3,5-8, 10-12 |
| Y | Masao KOBAYASHI, "Keiko Johosei Seizai no Ryushi Sekkei", Ryushi Sekkei to Seizai Gijutsu, Kabushiki Kaisha Yakuji Jihosha, 30 October, 1993 (30.10.93), pages 27 to 32 | 1-12 |
| A | Edited by Hitoshi SEZAKI, Drug Delivery System, Nankodo Co., Ltd., 15 April, 1986 (15.04.86), pages 109 to 135 | 1-12 |

Form PCT/ISA/210 (continuation of second sheet) (April 2005)

**INTERNATIONAL SEARCH REPORT**

International application No.

PCT/JP2006/301401

Continuation of A. CLASSIFICATION OF SUBJECT MATTER
  (International Patent Classification (IPC))

*A61P7/12*(2006.01), *C07D233/60*(2006.01)

          (According to International Patent Classification (IPC) or to both national
          classification and IPC)

Form PCT/ISA/210 (extra sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 7215943 A

- WO 0134147 A1

**Non-patent literature cited in the description**

- *Bioorg. Med. Chem.,* 1997, vol. 7, 1151-116